# EUROPÄISCHE PATENTSCHRIFT

(11) **EP 0 610 196 B1**
(45) Veröffentlichungstag und Bekanntmachung des Hinweises auf die Patenterteilung: **03.07.1996**
(21) Anmeldenummer: 92909280.7
(22) Anmeldetag: 30.04.1992
(51) Int. Cl.: C12Q 1/68

(54) **VERFAHREN ZUR DIAGNOSE VON NICHT-INSULIN-ABHÄNGIGEM DIABETES MELLITUS**
PROCESS FOR DIAGNOSING NON-INSULIN-DEPENDENT DIABETES MELLITUS
PROCEDE DE DIAGNOSTIC DU DIABETE SUCRE NON INSULINO-DEPENDANT

(30) Priorität: 02.05.1991 DE 4114365
(43) Veröffentlichungstag der Anmeldung: 17.08.1994
(73) Patentinhaber: Max-Planck-Gesellschaft zur Förderung der Wissenschaften e.V., D-37073 Göttingen (DE)
(72) Erfinder: SEEDORF, Luitgard, D-8000 München 70 (DE); HÄRING, Hans-U., D-8000 München 40 (DE); ULLRICH, Axel, D-8000 München 22 (DE)
(74) Vertreter: Wright, Simon Mark
(86) Internationale Anmeldenummer: EP9200949
(87) Internationale Veröffentlichungsnummer: WO9219769

(56) Entgegenhaltungen:
- EP-A- 0 314 500
- US-A- 4 965 189
- THE EMBO JOURNAL, vol. 9, no. 8, 1990, Eynsham, Oxford (GB); L. MOSTHAF et al., pp. 2409-2413/
- BIOCHEMICAL & BIOPHYSICAL RESEARCH COMMUNICATIONS, vol. 159, no. 1, 28 February 1989, Duluth, MN (US); S. SEINO et al., pp. 312-316/
- NATURE, vol. 313, 28 February 1985, London (GB); A. ULLRICH et al., pp. 756-761/
- PROCEEDINGS OF THE NATL. ACADEMY OF SCIENCES USA, vol. 88, June 1991, Washington, DC (US);4728 - 4730; L. MOSTHAF et al., pp. 4728-4730/

## Beschreibung

Die Erfindung betrifft ein Verfahren zur Diagnose von nicht-Insulin-abhängigem Diabetes mellitus oder einer genetisch bedingten Veranlagung dafür.

Diabetes mellitus vom Typ 2 (nicht-Insulin-abhängiger Diabetes mellitus, NIDDM) hat in den letzten Jahrzehnten in der Bevölkerung stark zugenommen. Es besteht daher ein großes Interesse daran, eine leicht durchführbare Möglichkeit bereitzustellen, diesen Diabetes frühzeitig zu diagnostizieren, bzw. möglichst schon vor dem tatsächlichen Ausbruch der Krankheit eine genetisch bedingte Veranlagung hierfür verläßlich feststellen zu können.

Aufgabe der vorliegenden Erfindung war es daher, ein Verfahren bereitszustellen, das es ermöglicht, schnell und präzise zu bestimmen, ob die genetischen Grundlagen für die Krankheit in einem Patienten vorliegen.

Gelöst wird diese Aufgabe durch das erfindungsgemäße Verfahren zur Diagnose von nicht-Insulin-abhängigem Diabetes mellitus oder einer genetisch bedingten Veranlagung dafür, bei dem man Zellen, insbesondere Skelettmuskelzellen von Patienten auf Anwesenheit von humanem Insulin-Rezeptor vom Typ B (HIR-B) untersucht.

Das erfindungsgemäße Verfahren beruht auf der überraschenden Erkenntnis, daß ganz spezifische Unterschiede im Vorhandensein der beiden Formen der Insulin-Rezeptoren (HIR-A, HIR-B) bei Diabetikern gegenüber Gesunden in den Zellen nachgewiesen werden können.

Die beiden Formen des Rezeptors sind bereits längere Zeit bekannt und Untersuchungen hierzu beispielsweise von Mosthaf et al., EMBO Journal, Vol. 9, Nr. 8, Seiten 2409-2413 (1990) ebenso wie in früheren Literaturstellen, auf die dort hingewiesen wird, insbesondere Ebina et al., Proc.Natl.Acad.Sci. USA 84 (1987), 704-708; Ullrich et al., Nature 313 (1985), 756-761; Seino, S. und Bell, F.I., Biochem.Biophys.Res. Commun. 159 (1989), 312-316 und Yarden, Y. und Ullrich, A., Annu.Rev.Biochem. 57 (1988), 443-478 beschrieben. Hierbei wurde festgestellt, daß zwei verschiedene Rezeptorformen, benannt A und B, vorkommen, die beide aus durch Disulfidbrücken verbundenen gleichen α- und β-Untereinheiten bestehen, wobei jedoch ein Unterschied dahingehend besteht, daß die α-Untereinheit des B-Typs einen Einschub von 12 Aminosäuren in der C-terminalen Region aufweist. Da nur ein einziges Gen für einen Insulin-Rezeptor identifiziert werden konnte, lag die Vermutung nahe, daß es sich bei der Entstehung der beiden unterschiedlichen Formen A und B um einen posttranskriptionellen Mechanismus handelt. Es wurde weiter festgestellt, daß das 12 Aminosäuren-Insert der B-Form von einem getrennten Exon kodiert wird und die beiden Formen durch alternatives Splicen des Primärtranskripts entstehen. Es wurde hierzu ebenfalls festgestellt, daß das Vorliegen von HIR-A und HIR-B RNA sowohl mengenmäßig als auch prinzipiell gewebeabhängig ist. Man fand außerdem, daß die beiden Rezeptorformen unterschiedliche Bindungscharakteristika für Insulin aufweisen.

Im Rahmen der vorliegenden Erfindung wurde nun festgestellt, daß in Skelettmuskelzellen ganz spezifisch bei NIDDM-Patienten oder Personen mit einer genetisch bedingten Veranlagung hierzu etwa gleiche Mengen an Typ A und Typ B des humanen Insulin-Rezeptors vorliegen, während bei gesunden Menschen in den gleichen Zellen lediglich Rezeptor vom Typ A aufgefunden wird. Dies im Unterschied zu einigen anderen Zelltypen, bei denen auch beim gesunden Menschen beide Rezeptor-Typen vorliegen können, wie aus den oben bereits genannten Literaturstellen zu ersehen ist. Im Unterschied zu den Gewebezellen wird beispielsweise bei menschlichen Blutzellen kein Unterschied sichtbar zwischen nicht-diabetischen Personen und NIDDM-Patienten. In beiden Fällen liegt dort lediglich Rezeptor vom Typ A vor, während Rezeptor vom Typ B nicht nachgewiesen werden kann.

Im erfindungsgemäßen Verfahren können prinzipiell alle Methoden zur Anwendung kommen, die eine Unterscheidung der beiden Rezeptor-Typen in den Gewebezellen erlauben. Gewebezellen können hierzu in an sich bekannter Weise, insbesondere durch Biopsie vom Patienten erhalten werden.

In einer bevorzugten Ausgestaltung des erfindungsgemäßen Verfahrens wird so vorgegangen, daß man die Untersuchung auf Anwesenheit des Rezeptors vom Typ B in Skelettmuskelzellen mit Hilfe spezifischer Oligonukleotide durchführt, bei deren Verwendung in einer PCR-Amplifikationsreaktion von aus RNA der Zellen erhaltener cDNA für HIR-A und B kodierende DNA amplifiziert wird. Hierzu wird aus Skelettmuskelgewebe zuerst in an sich bekannter Weise die RNA präpariert, aus der dann wiederum in ebenfalls an sich bekannter Weise eine cDNA synthetisiert wird.

Die RNA kann dabei insbesondere nach einer eigens entwickelten Methode präpariert werden, die auch aus kleinsten Mengen an Gewebe (20-40 mg) ausreichende Mengen an mRNA liefert und in Beispiel 1b) beschrieben ist.

Die erhaltene A⁺RNA wird dann für die cDNA-Synthese verwendet. Die cDNA wird vorzugsweise spezifisch mit einem Oligonukleotid geprimt, welches der Sequenz des Komplementärstrangs der Nukleotide 2858 bis 2879 der humanen Insulin-Rezeptor A cDNA-Sequenz entspricht (Numerierung gemäß Ullrich et al. in Nature 313 (1985) 756-761). Die Synthese des ersten Strangs der cDNA wird wie beispielsweise bei Sambrook et al. (1989; CSH Laboratory Press, 2nd edition) beschrieben durchgeführt.

Die cDNA wird dann ohne weitere Aufreinigung einer Vielzahl von (z.B. etwa 40) Zyklen der Polymerase-Kettenreaktion unterworfen. Als Primer dienen vorzugsweise Oligonukleotide, die die Insertionsstelle für die beim Rezeptor B eingeschobenen 36 Nukleotide flankieren. Sie sind also so gewählt, daß ein Oligonukleotid mit der upstream und das zweite Oligonukleotid mit der downstream von der Insertionsstelle gelegenen Sequenz hybridisieren. Dabei ist die Sequenz des ersten Oligonukleotids identisch mit der Sequenz der RNA (statt U aber T), das zweite Oligonukleotid ist hingegen identisch mit der Sequenz des Komplementärstrangs. Das erste Oligonukleotid entspricht in einer bevorzugten Ausführungsform der Erfindung den Nukleotiden 2136 bis 2257 der humanen Insulin-Rezeptor A cDNA, das zweite der Komplementärsequenz der Nukleotide 2327 bis 2348. Die Reaktion wurde durchgeführt wie bei Mosthaf et al. beschrieben (EMBO J. 9, 2409 bis 2413, 1990) und ergibt bei Verwendung der bevorzugten Oligonukleotide spezifische DNA-Fragmente von 112 (HIR-A) bzw. 148 (HIR-B) Basenpaaren.

Bei Anwesenheit von cDNA beider Rezeptorvarianten werden dann zwei Amplifikationsprodukte bei der PCR-Reaktion erhalten, liegt lediglich mRNA vom Typ A vor, erhält man nur ein Amplifikationsprodukt.

Das Vorliegen eines Amplifikationsproduktes für HIR-A kann sowohl als positive Kontrolle bei der Untersuchung verwendet werden, ebenso können die Mengen von HIR-B im Vergleich zu HIR-A dadurch abgeschätzt werden, wobei bei Anwesenheit deutlicher Mengen von HIR-B im Vergleich zu HIR-A vom Vorliegen von nicht-Insulin-abhängigem Diabetes mellitus oder einer genetisch bedingten Veranlagung dafür ausgegangen werden muß.

Das erfindungsgemäße Verfahren ermöglicht es in einfacher Weise durch die schnelle Durchführung einer PCR-Reaktion von aus Gewebe isolierter RNA und daraus wiederum gewonnener cDNA festzustellen, ob in dem Gewebe auch HIR-B RNA gebildet wird, was auf das Vorliegen von NIDDM bzw. einer Veranlagung dafür hindeutet, oder aber ob lediglich HIR-A RNA vorhanden ist, woraus sich schließen ließe, daß weder NIDDM noch eine besondere Veranlagung hierfür bei dem Patienten vorliegt.

Ein weiterer Gegenstand der Erfindung sind Oligonukleotide, die die bevorzugt verwendeten Primersequenzen für sowohl die cDNA-Synthese aus RNA von Gewebezellen als auch die PCR-Reaktion umfassen.

Die vorliegende Erfindung wird durch das folgende Beispiel in Verbindung mit den Figuren weiter erläutert.
- Figur 1: zeigt hierbei die elektrophoretisch aufgetrennten PCR-Produkte von gesunden Personen bzw. von Patienten mit NIDDM, erhalten ausgehend von Skelettmuskelgewebe.
- Figur 2: zeigt einen Vergleichsversuch, bei dem Blutzellen entsprechend untersucht wurden.

### Beispiel

### a) Gewebeproben

Skelettmuskelproben (Musculus gastroenaemius) wurden von diabetischen (N = 10) und nicht-diabetischen (N = 6) 60 bis 80 Jahre alten Patienten nach Beinamputationen bzw. Biopsie erhalten. Alle Proben wurden sofort nach der Operation in kleine Stücke von 0,3 g geschnitten, in flüssigem Stickstoff gefroren und bei -80°C aufbewahrt. Die Muskelproben wurden einer Qualitätskontrolle unterworfen unter Beurteilung mit folgenden Mitteln:
1. Mikroskopie, Elektronenmikroskopie und histochemische Qualität des Gewebes,
2. enzymatische Aktivität von Lactatdehydrogenase EC 1.1.1.27 (LDH), Phosphofructokinase EC 2.7.1.11 (PFK), Phosphoglyceratkinase EC 2.7.2.3 (PGK) und Phosphoglucomutase EC 2.7.5.1 (PGM) und
3. Untersuchung auf Nicht-Collagenprotein (NCP) im normalen Bereich des Muskelgewebes.

Die Methodologie und die morphologischen und enzymatischen Bestimmungen sind in Obermaier-Kusser et al., J.Biol.Chem. 164 (1989), 9497-9503 dargestellt.

Blutproben (5 ml) wurden von 5 NIDDM-Patienten und 5 Kontrollen am Morgen nach einer 12-stündigen Fastenperiode genommen, mit EDTA vermischt, um Gerinnung zu verhindern und sofort danach in flüssigem Stickstoff gefroren.

### b) RNA-Präparation

Dazu wurden 0,02 bis 1 g gefrorenes Muskelgewebe mit einem Polytron in 1 ml Puffer A (0,5 M NaCl, 1 % SDS, 10 mM Tris-HCl pH 7,5, 1 mM EDTA, 200 µg/ml Proteinase K) homogenisiert und anschließend 1 Stunde bei 37°C inkubiert. Nach Zugabe von 20 bis 50 µl einer oligo(dT)Cellulose Suspension (in Puffer A) wurde die Probe bei 37°C für 1 Stunde geschüttelt. Die an die oligo(dT)Cellulose gebundene mRNA wurde durch eine kurze Zentrifugation in einer Tischzentrifuge pelletiert, 2 x mit Puffer B (0,5 M NaCl, 0,2 % SDS, 10 mM Tris-HCl pH 7,5, 1 mM EDTA, 1 mM PMSF) gewaschen und anschließend 1 x mit Puffer C gewaschen (0,5 M NaCl, 10 mM Tris HCl pH 7,5, 1 mM EDTA). Die mRNA wurde mit 3 Waschschritten von je 20 µl H₂O von der oligo(dT)Cellulose eluiert, anschließend 5 Minuten auf 68°C erhitzt und schnell auf Eis abgekühlt.

Zur Isolierung von RNA aus Blut wurden 5 ml Blut mit einer 2x Guanidinthiocyanat-Lösung verdünnt zu einer Endkonzentration, homogenisiert und mit LiCl gefällt, wie bei Ullrich et al., Science 196 (1977), 1313 beschrieben.

### c) cDNA-Synthese und Polymerase-Kettenreaktion (PCR)

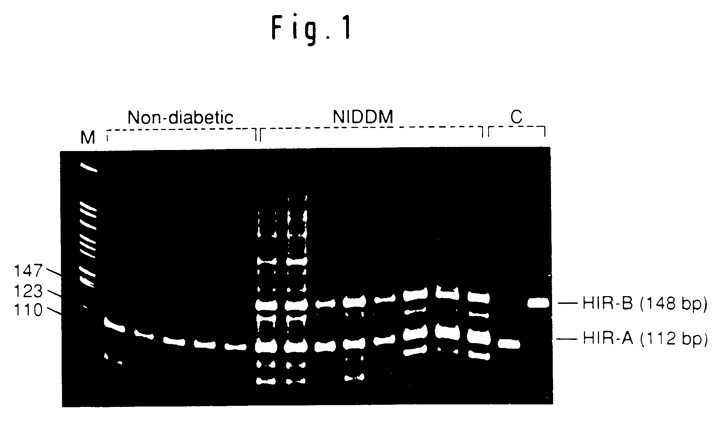

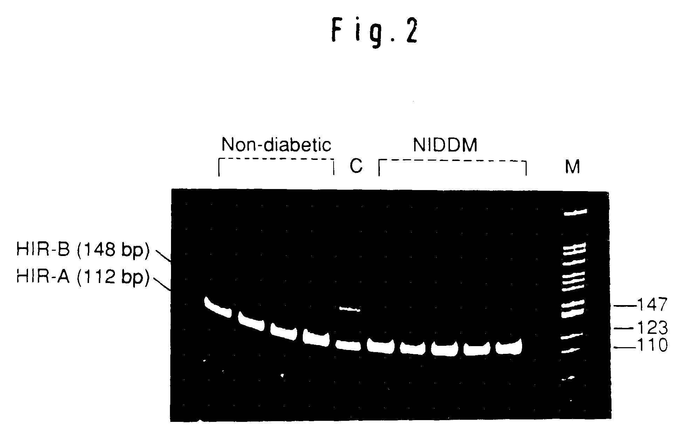

5 bis 10 µg Gesamt-RNA wurden verwendet für die cDNA-Synthese. Die cDNA wurde spezifisch geprimt mit einem Oligonukleotid, das die Nukleotide 2858 bis 2879 der humanen Insulinrezeptor A-Sequenz (Ullrich et al., Nature 313 (1985), 756 bis 761) umfaßt. Erststrang-cDNA-Synthese wurde im wesentlichen gemäß Sambrook et al., CSH Laboratory Press, 2nd Edition (1989) in einem Gesamtvolumen von 20 µl von 50 mmol/l KCl, 10 mmol/l Tris/HCl, pH 8,3, 4 mmol/l MgCl₂, 1 mmol/l dNTPs, 10 µg/ml BSA (Rinderserumalbumin), 50 pmol Primer und 500 Units Reverser Transkriptase durch Inkubation für 1 Stunde bei 37°C durchgeführt. Das Reaktionsprodukt wurde ohne weitere Reinigung 40 Zyklen einer PCR-Reaktion unterworfen. Die PCR wurde durchgeführt, wie in Mosthaf et al., EMBO J. 9 (1990), 2409-2413 beschrieben, unter Verwendung von Oligonukleotiden, die die 12 Aminosäuren-Insertionsstelle flankieren. Die verwendeten Oligonukleotide repräsentierten die Nukleotide 2136-2257 und die Komplementärsequenz der Nukleotide 2327-2348 der humanen Insulin-Rezeptor-Sequenz (Ullrich, supra) und führten zur Bildung von spezifischen Fragmenten von 112 (HIR-A) und 148 (HIR-B) Basenpaaren (siehe Figur 1, Figur 2). In Figur 1 ist die PCR-Analyse von HIR-A- und HIR-B-Sequenzen aus Muskelproben von nicht-diabetischen und diabetischen (NIDDM) Personen gezeigt. Die Kontrollspur (C) enthält PCR-Fragmente, die erhalten wurden aus klonierter HIR-A oder HIR-B-cDNA. Fragmente, die nicht auf gleicher Höhe mit den HIR-A- oder HIR-B-Kontrollen wandern, stellen unspezifische oder einzelsträngige Nebenprodukte der PCR-Reaktion dar. In Figur 2 ist die PCR-Analyse von HIR-A und HIR-B-Sequenzen in humanen Blutzellen gezeigt. Die Kontrollspur (C) enthält eine Mischung von PCR-Fragmenten, die erhalten wurden aus klonierter HIR-A- und HIR-B-cDNA. Die Spur (M) enthält in beiden Figuren Größenmarker. Aus diesem Beispiel wird deutlich, daß bei Patienten mit NIDDM mit Hilfe einer PCR-Analyse von Skelettmuskel es möglich ist, die Anwesenheit von HIR-B nachzuweisen und damit hier einen deutlichen Unterschied zu gesunden Personen aufzuzeigen. In Blutzellen ist hier keinerlei Unterscheidung möglich.

## Patentansprüche

1. Verfahren zur Diagnose von nicht-Insulin-abhängigem Diabetes mellitus oder einer genetisch bedingten Veranlagung dafür,
**dadurch gekennzeichnet,** daß man Skelettmuskelzellen der jeweiligen Personen auf Anwesenheit von humanem Insulin-Rezeptor vom Typ B (HIR-B) untersucht.

2. Verfahren nach Anspruch 1,
**dadurch gekennzeichnet,** daß man die Untersuchung mit Hilfe spezifischer Oligonukleotide durchführt, bei deren Verwendung als Primer in einer PCR-Amplifikationsreaktion von aus RNA des Gewebes erhaltener cDNA zwischen HIR-A und HIR-B RNA unterschieden werden kann.

3. Verfahren nach Anspruch 2,
**dadurch gekennzeichnet,** daß man zur Erzeugung von cDNA aus RNA des Gewebes einen Oligonukleotidprimer verwendet, welcher zu den Nukleotiden 2858 bis 2879 der humanen Insulin-Rezeptor A cDNA-Sequenz komplementär ist.

4. Verfahren nach Anspruch 2 oder 3,
**dadurch gekennzeichnet,** daß man ein erstes Oligonukleotid verwendet, das komplementär ist zu der Sequenz der Nukleotide 2327 bis 2348 der cDNA-Sequenz, welche für den humanen Insulin-Rezeptor A codiert und ein zweites Oligonukleotid verwendet, das der Sequenz der Nukleotide 2136 bis 2257 der cDNA-Sequenz, welche für den humanen Insulin-Rezeptor Typ A codiert, entspricht.

5. Verfahren nach Ansprüche 1, 2, 3 oder 4 dadurch gekennzeichnet daß die Skelettmuskelzellen durch eine Biopsie erhalten werden.

6. Verwendung eines Oligonukleotids als Primer in einem Verfahren gemäß einem der Ansprüche 2 bis 5, dadurch gekennzeichnet, daß es eine Sequenz umfaßt, die komplementär ist zu den Nukleotiden 2327 bis 2348 der humanen Insulin-Rezeptor A cDNA-Sequenz.

7. Verwendung eines Oligonukleotids als Primer in einem Verfahren gemäß einem der Ansprüche 2 bis 5, dadurch gekennzeichnet, daß es eine Sequenz umfaßt, die der Sequenz der Nukleotide 2136 bis 2257 der humanen Insulin-Rezeptor A cDNA-Sequenz entspricht.

8. Verwendung eines Oligonukleotids als Primer in einem verfahren nach einem der Ansprüche 2 bis 5, zur Erzeugung von cDNA aus RNA von Skelettmuskelzellen, dadurch gekennzeichnet, daß es eine Sequenz umfaßt, die zu der Sequenz der Nukleotide 2858 bis 2879 der humanen Insulin-Rezeptor A cDNA-Sequenz komplementär ist.

## Claims

1. A method for the diagnosis of non-Insulin dependent Diabetes Mellitus or a genetically determined predisposition therefor, characterised in that skeletal muscle cells of given people are examined for the presence of the human Insulin Receptor of type B (HIR-B).

2. A method according to claim 1, characterised in that the examination is carried out with the help of special oligonucleotides, which can be used as primers in a PCR amplification reaction of cDNA derived from RNA of a tissue so as to distinguish between HIR-A and HIR-B RNA.

3. A method according to claim 2 characterised in that an oligonucleotide primer is used in producing cDNA from the RNA of a tissue which is complementary to nucleotides 2858 to 2879 of the human insulin receptor A cDNA sequence.

4. A method according to claim 2 or claim 3 characterised in that a first oligonucleotide is used which is complementary to the sequence of nucleotides 2327 to 2348 of a cDNA sequence which codes for human insulin receptor A and a second oligonucleotide is used which corresponds to the sequence of nucleotides 2136 to 2257 of a cDNA sequence coding for human insulin receptor type A.

5. A method according to claim 1, 2, 3 or 4, characterised in that the skeletal muscle cells are obtained through a biopsy.

6. The use of an oligonucleotide as a primer in a method according to one of claims 2 to 5, characterised in that it comprises a sequence that is complementary to nucleotides 2327 to 2348 of the human insulin receptor A cDNA sequence.

7. The use of an oligonucleotide as a primer in a method according to one of claims 2 to 5, characterised in that it comprises a sequence which corresponds to the cDNA sequence of nucleotides 2136 to 2257 of human insulin receptor A.

8. The use of an oligonucleotide as a primer in a method according to one of claims 2 to 5 in the production of cDNA from RNA of skeletal muscle cells characterised in that it comprises a sequence which is complementary to the sequence of nucleotides 2858 to 2879 of the human insulin receptor A cDNA sequence.

## Revendications

1. Procédé pour le diagnostic du diabète sucré non insulino-dépendant ou d'une prédisposition génétique à celui-ci, caractérisé en ce que l'on examine des cellules de muscles squelettiques des patients, en présence du récepteur insulinique humain de type B (HIR-B).

2. Procédé suivant la revendication 1, caractérisé en ce que l'on réalise l'examen au moyen d'oligonucléotides spécifiques dont l'utilisation comme amorces dans une amplification en chaîne par polymérase - ACP de l'ADNc obtenu à partir de l'ARN du tissu peut différencier l'ARN de HIR-A et l'ARN de HIR-B.

3. Procédé suivant la revendication 2, caractérisé en ce que l'on utilise pour la production de l'ADNc à partir de l'ARN du tissu, une amorce oligonucléotidique qui est complémentaire aux nucléotides 2858 à 2879 de la séquence de l'ADNc du récepteur insulinique humain A.

4. Procédé suivant la revendication 2 ou 3, caractérisé en ce que l'on utilise un premier oligonucléotide qui est complémentaire à la séquence des nucléotides 2327 à 2348 de la séquence d'ADNc qui code pour le récepteur insulinique humain A et un deuxième oligonucléotide qui correspond à la séquence des nucléotides 2136 à 2257 de la séquence d'ADNc qui code pour le récepteur insulinique humain de type A.

5. Procédé suivant les revendications 1, 2, 3 ou 4, caractérisé en ce que l'on se procure les cellules de muscles squelettiques par biopsie.

6. Utilisation d'un oligonucléotide comme amorce dans un procédé suivant l'une quelconque des revendications 2 à 5, caractérisé en ce qu'il comprend une séquence qui est complémentaire aux nucléotides 2327 à 2348 de la séquence d'ADNc du récepteur insulinique humain A.

7. Utilisation d'un oligonucléotide comme amorce dans un procédé suivant l'une quelconque des revendications 2 à 5, caractérisé en ce qu'il comprend une séquence qui correspond à la séquence des nucléotides 2136 à 2257 de la séquence d'ADNc du récepteur insulinique humain A.

8. Utilisation d'un oligonucléotide comme amorce dans un procédé suivant l'une quelconque des revendications 2 à 5, pour la production d'ADNc à partir d'ARN de cellules de muscles squelettiques, caractérisé en ce qu'il comprend une séquence qui est complémentaire à la séquence des nucléotides 2858 à 2879 de la séquence d'ADNc du récepteur insulinique humain A.
